(19) Europäisches Patentamt
European Patent Office
Office européen des brevets

(11) Numéro de publication : **0 480 983 B1**

# (12) FASCICULE DE BREVET EUROPEEN

(45) Date de publication du fascicule du brevet : **08.12.93 Bulletin 93/49**

(51) Int. Cl.$^5$ : **A61K 35/78,** C11C 3/00

(21) Numéro de dépôt : **90910774.0**

(22) Date de dépôt : **02.07.90**

(86) Numéro de dépôt international : **PCT/FR90/00494**

(87) Numéro de publication internationale : **WO 91/00101 10.01.91 Gazette 91/02**

## (54) UTILISATION DE LIPIDES PEROXYDES POUR LA PREPARATION D'UN MEDICAMENT DESTINE AU TRAITEMENT PAR APPLICATION LOCALE DES INSUFFISANCES CIRCULATOIRES.

(30) Priorité : **03.07.89 FR 8908883**

(43) Date de publication de la demande : **22.04.92 Bulletin 92/17**

(45) Mention de la délivrance du brevet : **08.12.93 Bulletin 93/49**

(84) Etats contractants désignés : **AT BE CH DE DK ES FR GB IT LI LU NL SE**

(56) Documents cités :
**EP-A- 0 117 962**
**EP-A- 0 225 831**
**EP-A- 0 225 832**
**EP-A- 0 225 833**
**EP-A- 0 226 506**

(56) Documents cités :
**EP-A- 0 293 535**
**FR-A- 2 461 744**
**FR-A- 2 539 142**
**FR-A- 2 591 112**
**FR-M- 2 330**

(73) Titulaire : **LABORATOIRES CARILENE, SA**
**7, rue du Chant des Oiseaux**
**F-78360 Montesson (Les Yvelines) (FR)**

(72) Inventeur : **DESJONQUERES, Stéphane**
**7, avenue Marcellin-Berthelot**
**F-78360 Montesson (FR)**

(74) Mandataire : **Portal, Gérard et al**
**Cabinet Beau de Loménie 158, rue de l'Université**
**F-75340 Paris Cédex 07 (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



Jouve, 18, rue Saint-Denis, 75001 PARIS

## Description

La présente invention concerne généralement une nouvelle indication thérapeutique de produits connus, et a essentiellement pour objet l'utilisation de lipides peroxydés pour la préparation d'un médicament destiné au traitement par application locale des insuffisances circulatoires, et notamment de l'asthénie sexuelle masculine.

On sait que de nombreuses personnes souffrent d'insuffisance circulatoire d'origine artérielle ou veineuse, souvent chronique.

Les insuffisances circulatoires ont parfois un caractère local et bénin se manisfestant par exemple par une sensation d'engourdissement douloureux du bout des doigts causée par un grand froid (onglée) ou encore de lourdeur au niveau des membres inférieurs.

Les insuffisances circulatoires peuvent également se manifester sous des formes beaucoup plus graves, liées à un arrêt de la circulation sanguine (ischémie) pouvant entrainer une nécrose localisée. Les ulcères artériels ou veineux de la jambe sont, par exemple, le résultat d'une telle insuffisance circulatoire, manifestation clinique d'une insuffisance d'irrigation des tissus superficiels et profonds par le flux sanguin.

De nombreux traitements ont été proposés jusqu'à ce jour pour remédier à ces insuffisances.

Ainsi, on a déjà préconisé l'utilisation de nombreux produits vasodilatateurs ou vasoprotecteurs par voie orale ou percutanée, souvent par perfusion.

Cependant, ces produits présentent de nombreux effets secondaires indésirables, de sorte que leur utilisation est réservée aux cas les plus sérieux d'insuffisance circulatoire.

En outre, il n'a jamais été proposé, jusqu'à présent, de produits pour la revascularisation des tissus, totalement exempts de toxicité et susceptibles d'être appliqués par voie locale, dont l'utilisation puisse être envisagée pour le traitement d'insuffisance circulatoire à tous les stades.

Il a été découvert, et ceci constitue le fondement de la présente invention, que certains lipides peroxydés présentent une très grande efficacité en application locale pour la revascularisation des tissus, et que ces produits n'entraînent aucun effet secondaire indésirable.

Ainsi, la présente invention a pour objet l'utilisation de lipides peroxydés, présentant un taux de peroxydation compris entre 50 et 200 milli-équivalents par kilo, de préference entre 50 et 150 milli-équivalents par kilo, pour la préparation d'une composition pharmaceutique destinée au traitement par application locale des insuffisances circulatoires.

Il a été observé qu'avec un taux de peroxydation intérieur à 50 milli-équivalents par kilo, on n'obtenait pas d'efficacité significative.

Par ailleurs, avec un taux de peroxydation supérieur à 200 milli-équivalents par kilo, il y avait génération de radicaux libres néfastes.

Les lipides peroxydés utilisés conformément à l'invention sont des produits connus préparés à partir de lipides par exemple par saturation en oxygène et exposition intensive et controlée aux rayons ultraviolets.

Ces produits ont notamment été décrits dans les documents BSM n° 2330 M, EP-A-293 535, FR-A-2 591 112 ; EP-A-225 831 ; EP-A-225 832 ; EP-A-225 833 ; EP-A-226 506 ; FR-A-2 461 744 ; FR-A-2 539 142 et EP-A-117 962.

Dans ces documents antérieurs, ces composés sont présentés comme susceptibles d'être utilisés dans le traitement de certaines affections, notamment en rhumatologie ou traumatologie, ou encore en tant que produit cicatrisant.

Cependant, l'enseignement de cet état de la technique ne comportait aucune indication, ou même une lointaine suggestion ve.s l'utilisation de ces produits pour la préparation d'une composition pharmaceutique destinée au traitement des insuffisances circulatoires.

D'une façon tout à fait surprenante et inattendue, on a constaté que l'application locale de ces produits était de nature à augmenter de façon remarquable, non seulement le flux sanguin superficiel, mais également le flux sanguin sous-cutané ou profond. En particulier, il a été constaté que ces produits provoquent un flux sanguin double du flux sanguin préalable à l'application du produit. Cette augmentation de flux sanguin est nettement supérieure à celle obtenue avec des produits vasodilatateurs classiques employés en perfusion et résulte, semble-t-il, d'un mécanisme d'action différent.

Les lipides peroxydés susceptibles d'être utilisés conformément à l'invention peuvent être de nature chimique très variée, mais doivent nécessairement présenter un taux de peroxydation, mesuré par la méthode AFNOR, compris entre 50 et 200 milli-équivalents par kilo, et de préférence entre 50 et 150 milli-équivalents par kilo.

Selon un mode de réalisation particulier, ces lipides présentent avantageusement une teneur en glycérides oxydés comprise entre 5 et 40 %.

Conformément à l'invention, on utilise préférentiellement comme lipide peroxydé au moins un peroxyde

obtenu par peroxydation de lipides d'origine végétale, par exemple sous forme d'au moins une huile végétale naturelle. De préférence, ces huiles sont choisies par l'huile d'amande douce, l'huile de noisette, l'huile d'arachide, l'huile de mais, l'huile de pépin de raisin, l'huile de sésame et l'huile de carthame.

Selon un mode de réalisation particulier de l'invention, on utilise des lipides peroxydés constitués principalement ou majoritairement de triglycérides répondant à la formule générale :

$$
\begin{array}{l}
CH_2-O-R \\
| \\
CH-O-R \\
| \\
CH_2-O-R
\end{array}
$$

dans laquelle les radicaux R sont majoritairement représentés par les acides octadécanoiques et octadécanoiques peroxydés.

Selon une caractéristique particulière de l'invention, ces produits sont utilisés pour la préparation d'une composition pharmaceutique destinée à être appliquée localement, et se présentent de manière générale sous la forme d'une crème, d'un gel. On peut également envisager des compositions se présentant sous forme de liquide, ou encore sous forme de capsule molle contenant les liquides peroxydés.

Une composition pharmaceutique ainsi préparée comprend, exprimée en pourcentages pondéraux, 2 à 99 % de lipides peroxydés, environ 1 % de parfum, ainsi que des excipients pharmaceutiquement acceptables et de l'eau déminéralisée.

Avantageusement, cette composition pharmaceutique se présente sous forme d'une crème contenant :

- alcool cétylique 2,0 %
- acide stéarique 5,0 %
- lipides peroxydés 25,0 %
- silicones 0,5 %
- propylèneglycol 3,0 %
- triéthanolamine 0,2 %
- parfum 0,8 %
- émulsionnant non ionique 6,0 %
- antimicrobien 0,3 %
- eau déminéralisée q.s.q. 100

L'effet d'augmentation du flux sanguin des composés utilisés conformément à l'invention a été déterminé expérimentalement à partir de mesures de vitesse circulatoire dans des vaisseaux superficiels par Doppler et de mesures de la circulation globale superficielle et profonde par rhéopléthysmographie.

Les exemples suivants permettront d'illustrer la présente invention.

Exemple 1 :

Préparation d'une composition pharmaceutique à base de lipides peroxydés, provenant d'une huile de carthame vierge disponible dans le commerce

De l'huile de carthame vierge disponible dans le commerce présentant les caractéristiques principales suivantes :

- indice de peroxyde 18,70 meq/kg
- glycéride oxydé 4,18 %
- viscosité à 50°C 23,2 centistoke

est soumise à un traitement de peroxydation comme décrit dans le document FR-A-2 461 744.

Ce procédé consiste à stocker l'huile de Carthame dans une cuve en inox. Cette cuve est coiffée d'une hotte pivotante capable de collecter les vapeurs et servant de support à une lampe à rayonnement ultra-violet, par exemple PHILIPS type HP 4 de 125 W.

Cette cuve est alimentée en continu par une pompe à air par exemple à un débit de 30 l/minute pour obtenir un effet de barbotage. La cuve est également chauffée par des résistances alimentées en courant électrique de façon à maintenir une température de 80 à 100°C.

L'huile est ainsi maintenue pendant une durée de 20 à 35 heures selon l'origine des lipides, notamment 25 heures pour l'huile de Carthame considérée dans cet exemple, afin d'obtenir un taux de peroxydation conforme à l'invention.

L'huile ainsi obtenue après peroxydation est stockée dans un réservoir de stockage qui reçoit de l'huile de carthame peroxydée pouvant provenir de plusieurs cuves de peroxydation.

L'huile de carthame peroxydée obtenue par le procédé rappelé ci-dessus présente les caractéristiques principales suivantes :

- indice de peroxyde 149,0 meq/kg
- glycéride oxydé 11,30 %
- viscosité à 50°C 25,9 centistoke

A partir de cette huile peroxydée, on peut préparer des capsules à paroi molle encapsulant l'huile de carthame peroxydée et libérant l'huile de carthame peroxydée lors d'une application topique, par massage.

Pour ce faire, on peut encapsuler l'huile de carthame peroxydée par tous procédés classiques d'encapsulation dans une capsule molle dont la tunique est réalisée principalement avec un mélange gélatine-eau/glycérine, la composition respective du contenu de la capsule molle et de la tunique de la capsule étant la suite par exemple pour un lot de 635,415 kg permettant de préparer 600 000 capsules en multiple et sous-multiple :

A - Contenu des capsules molles

| | |
|---|---|
| - huile de carthame peroxydée.... | 136,800 kg |
| - parfum anis.................... | 3,00 kg |
| - silice colloïdale............... | 10,200 kg |
| | 150,000 kg |

B - Tunique (paroi des capsules molles)

| | |
|---|---|
| - gélatine...................... | 211,796 kg |
| - glycérine..................... | 106,357 kg |
| - eau purifiée.................. | 164,147 kg |
| - bioxyde de titane............ | 2,414 kg |
| | 485,415 kg |

La machine utilisée permet par exemple de préparer des capsules molles de 460 mg ayant ainsi la composition suivante :

| Contenu | | mg/capsule |
|---|---|---|
| Huile de carthame peroxydée.......... | | 228,000 mg |
| Parfum anis réf. 9/500918............ | | 5,000 mg |
| Silice colloïdale.................... | | 17,000 mg |
| Pour un contenu de................... | | 250,000 mg |
| Tunique.............................. | | 210,000 mg ± 10 % |
| Bioxyde de titane.................... | 0,75 % | |
| Mélange gélatine-eau/glycérine (67/33) | q.s. % | |
| pour une capsule de.................. | | 460,000 mg ± 5 % |

Exemple 2 :

Mesure de la circulation profonde et périphérique par rhéopléthysmographie

a. Principe de la mesure

L'essai est effectué sur une surface de 10 x 10 cm au niveau des mollets (5 cm au-dessous de l'extrémité inférieure de la rotule).

Ces zones sont enduites de quelques gouttes d'huile de carthame peroxydée pure obtenue à l'exemple 1, par massage léger.

Les mesures sont faites à T=0 (avant application des produits) et T=5 mn après l'application de la composition pharmaceutique, une fois sur le mollet droit et une fois sur le mollet gauche.

Deux électrodes destinées à mesurer des variations de volume sont placées aux extrémités supérieure et inférieure.

Un brassard est placé au niveau de la cuisse. Il est gonflé à une pression de 5 cm de Hg, de manière à permettre l'arrivée du sang au niveau du membre examiné, tout en empêchant le "retour veineux".

On enregistre les variations de volume de ce segment de membre. Lors de l'occlusion, il se produit une augmentation jusqu'à une valeur maxima. Cette variation de volume initial traduit l'augmentation du flux artériel.

Lorsque la valeur maxima est atteinte, le brassard est dégonflé. La vidange veineuse se produit avec une courbe de décroissance d'abord rapide, puis lente. L'angle correspondant à la pente d'ascension de la courbe de remplissage est étudié.

Un essai comparable est réalisé en l'absence de composition.

b. Résultat

La comparaison des courbes obtenues après application de l'huile de carthame peroxydée selon l'invention, avec les courbes obtenues en l'absence de composition, a permis de constater une augmentation significative des volumes artériels dans le premier cas, traduite par une augmentation de l'angle de 0,5 à 1,4 et de 0,4 à 1,5.

En d'autres termes, les lipides peroxydés utilisés conformément à l'invention entraînent, par application locale, une augmentation du flux sanguin de plus de 100 %.

Exemple 3 :

Mesure des vitesses circulatoires par Doppler continu

a. Principe de la mesure

Pour effectuer cette étude, le sujet est assis les mains posées sur une surface plane.

Les artères radiales, cubitales, interosseuses et pulpaires sont étudiées au Doppler à l'état basal T=0. Les veines superficielles sont également examinées.

De l'huile de carthame peroxydée conforme à l'invention, pure, obtenue à l'exemple 1, est étalée sur les doigts des deux mains du sujet. Une étude des dix doigts étant impossible à réaliser en un seul temps, on a étudié, arbitrairement :

- le flux au niveau des dernières phalanges (flux pulpaire) de l'index et de l'annulaire droit ;
- le flux du pouce gauche (espace interosseux).

Une faible quantité de produit a été étalée sur chacun de ces segments en massant doucement.

Le sujet ne bouge pas les mains pendant les 30 min d'examen.

Les flux sont enregistrés après 1 min, 5 min, 10 min et 30 min. Des mesures sont également réalisées jusqu'à 6 h.

Les résultats obtenus ont montré une très nette augmentation des flux (augmentation des surfaces systolo-diastoliques) tant artériel que veineux dès la 1ère minute, avec un maintien au-delà de la 30e min. Les courbes ont également montré ce maintien jusqu'à 6 h.

Il n'a pas été observé de douleur cutanée sur les doigts enduits du produit. Il n'a pas été observé de rougeur, ce qui élimine une action révulsive du produit.

Exemple 4 :

Analyse de l'effet des lipides peroxydés sur des sujets atteints de la maladie de Raynaud.

On a cherché à mettre en évidence l'activité d'une composition à base de lipide peroxydé sur deux patientes atteintes de la maladie de Raynaud, en crise au moment de la mesure.

L'étude a été faite sur une main.

Les mesures ont été faites par un appareil Doppler.

Les courbes obtenues avec de l'huile de carthame peroxydée pure de l'exemple 1, ont montré une augmentation de la vascularisation avec réapparition progressive de l'onde de Ressaut. Dans le même temps, on a constaté une recoloration très nette des doigts traités avec cette composition entre la 15$^{e}$ et la 45$^{e}$ min. Cette constatation est en accord avec l'augmentation du flux pulpaire observé.

Exemple 5 :

Analyse de l'effet d'une composition à base de lipides peroxydés dans le traitement de l'asthénie sexuelle masculine.

On sait que l'asthénie sexuelle est souvent liée à un problème artériel veineux.

On a réalisé des mesures de variation du flux sanguin dans le réseau artériel et veineux nourricier du corps caverne avant et après application d'une capsule dosée à 460 mg obtenue à l'exemple 1, que l'on découpe pour libérer l'huile de carthame peroxydée qui est étalée en massant doucement.

Les mesures réalisées par Doppler pénien ont mis en évidence une augmentation significative des flux sanguins.

Une étude clinique sur un nombre significatif de sujets a montré que les lipides peroxydés conduisent à des résultats concluant sur la disfonction sexuelle après 45 j de traitement par application locale.

L'utilisation des lipides peroxydés conformes à l'invention permet également d'espérer une amélioration notable d'affections telles que les troubles trophiques des membres inférieurs, les insuffisances veineuses périphériques et artérielles, ou encore l'artériopathie diabétique.

## Revendications

1. Utilisation de lipides peroxydés, présentant un taux de peroxydation compris entre 50 et 200 milli-équivalents par kilo, de préférence entre 50 et 150 milli-équivalents par kilo, pour la préparation d'une composition pharmaceutique destinée au traitement par application locale des insuffisances circulatoires,

2. Utilisation selon la revendication 1, caractérisée en ce que la composition pharmaceutique est destinée au traitement de l'asthénie sexuelle masculine.

3. Utilisation selon la revendication 1 caractérisée en ce que la composition pharmaceutique est destinée au traitement de l'insuffisance circulatoire au niveau des mains.

4. Utilisation selon la revendication 1 caractérisée en ce que la composition pharmaceutique est destinée au traitement de l'insuffisance circulatoire au niveau des jambes.

5. Utilisation selon la revendication 1 caractérisée en ce que la composition pharmaceutique est destinée au traitement de la maladie de Raynaud.

6. Utilisation selon l'une des revendications 1 à 5 , caractérisée en ce que le lipide péroxydé précité est un peroxyde obtenu par peroxydation de lipides d'origine végétale, par exemple sous forme d'au moins une huile végétale naturelle.

7. Utilisation selon la revendication 6, caractérisée en ce que l'huile végétale naturelle est choisie parmi le groupe consistant de l'huile d'amande douce, d'huile de noisette, d'huile d'arachide, d'huile de maïs, d'huile de pépin de raisin, d'huile de sésame et d'huile de carthame.

8. Utilisation selon l'une des revendications 1 à 7 caractérisée en ce que les lipides peroxydés précités présentent avantageusement une teneur en glycérides oxydés comprise entre 5 et 40 %.

9. Utilisation selon l'une des revendications 1 à 8 caractérisée en ce que la composition pharmaceutique est préparée sous la forme d'une crème, d'un gel, d'un liquide ou de capsules molles contenant les lipides peroxydés.

10. Utilisation selon l'une quelconque des revendications 1 à 9, caractérisée en ce que la composition pha rmac eut i que précitée comprend entre 2 et 99 % en poids de lipides peroxydés.

11. Utilisation selon l'une des revendications 1 à 10 caractérisée en ce que la composition pharmaceutique

précitée comprend en outre environ 1 % de parfum, ainsi que des excipients pharmaceutiquement acceptables et de l'eau déminéralisée.

## Claims

1. Use of peroxodized lipids, having a degree of peroxidation of between 50 and 200 milliequivalents per kilogram, preferably of between 50 and 150 milliequivalents per kilogram, for the preparation of a pharmaceutical composition intended for the treatment of circulatory insufficiencies, by local application.

2. Use according to claim 1, characterized in that the pharmaceutical composition is intended for the treatment of male sexual asthenia.

3. Use according to claim 1, characterized in that the pharmaceutical composition is intended for the treatment of circulatory insufficiency in the hands.

4. Use according to claim 1, characterized in that the pharmaceutical composition is intended for the treatment of circulatory insufficiency in the legs.

5. Use according to claim 1, characterized in that the pharmaceutical composition is intended for the treatment of Raynaud's disease.

6. Use according to one of claims 1 to 5, characterized in that the afore-mentioned peroxidized lipid is a peroxide obtained by the peroxidation of lipids of vegetable origin, for example in the form of at least one natural vegetable oil.

7. Use according to claim 6, characterized in that the natural vegetable oil is selected from the group consisting of sweet-almond oil, hazelnut oil, peanut oil, maize oil, grapeseed oil, sesame oil and safflower oil.

8. Use according to one of claims 1 to 7, characterized in that the afore-mentioned peroxidized lipids advantageously have a content of oxidized glycerides of between 5 and 40%.

9. Use according to one of claims 1 to 8, characterized in that the pharmaceutical composition is prepared in the form of a cream, a gel, a liquid or soft capsules containing the peroxidized lipids.

10. Use according to any one of claims 1 to 9, characterized in that the afore-mentioned pharmaceutical composition comprises between 2 and 99% by weight of peroxidized lipids.

11. Use according to one of claims 1 to 10, characterized in that the afore-mentioned pharmaceutical composition also comprises about 1% of fragrance, as well as pharmaceutically acceptable excipients and demineralized water.

## Patentansprüche

1. Verwendung von Peroxidlipiden, die einen Peroxidationsgrad zwischen 50 und 200 Milliäquivalent pro Kilo, vorzugsweise zwischen 50 und 150 Milliäquivalent pro Kilo aufweisen, zur Herstellung einer pharmazeutischen Zusammensetzung, die für eine Behandlung von Kreislaufinsuffizienen durch lokale Anwendung bestimmt ist.

2. Verwendung nach Anspruch 1, dadurch gekennzeichnet, daß die pharmazeutische Zusammensetzung für die Behandlung maskuliner sexueller Asthenie bestimmt ist.

3. Verwendung nach Anspruch 1, dadurch gekennzeichnet, daß die pharmazeutische Zusammensetzung für die Behandlung einer Kreislaufinsuffizienz im Bereich der Hände bestimmt ist.

4. Verwendung nach Anspruch 1, dadurch gekennzeichnet, daß die pharmazeutische Zusammensetzung für die Behandlung einer Kreislaufinsuffizienz im Bereich der Beine bestimmt ist.

5. Verwendung nach Anspruch 1, dadurch gekennzeichnet, daß die pharmazeutische Zusammensetzung für die Behandlung des Raynaud-Syndroms bestimmt ist.

6. Verwendung nach einem der Ansprüche 1 bis 5, dadurch gekennzeichnet, daß das Peroxidlipid ein Peroxid ist, das durch Peroxidation von Lipiden pflanzlichen Ursprungs, beispielsweise in Form zumindest eines natürlichen Pflanzenöls, erhalten wird.

7. Verwendung nach Anspruch 6, dadurch gekennzeichnet, daß das natürliche Pflanzenöl aus der Gruppe bestehend aus Mandelöl, Nußöl, Erdnußöl, Maisöl, Traubenkernöl, Sesamöl und Safloröl ausgewählt ist.

8. Verwendung nach einem der Ansprüche 1 bis 7, dadurch gekennzeichnet, daß die Peroxidlipide vorteilhaft einen Gehalt an oxidierten Glyceriden zwischen 5 und 40 % aufweisen.

9. Verwendung nach einem der Ansprüche 1 bis 8, dadurch gekennzeichnet, daß die pharmazeutische Zusammensetzung in Form einer Creme, eines Gels, einer Flüssigkeit oder weicher Kapseln, welche die Peroxidlipide enthalten, hergestellt wird.

10. Verwendung nach einem der Ansprüche 1 bis 9, dadurch gekennzeichnet, daß die pharmazeutische Zusammensetzung zwischen 2 und 99 Gew.% Peroxidlipide umfaßt.

11. Verwendung nach einem der Ansprüche 1 bis 10, dadurch gekennzeichnet, daß die pharmazeutische Zusammensetzung außerdem ungefähr 1 % Parfum sowie pharmazeutisch annehmbare Exzipienten und entmineralisiertes Wasser umfaßt.